# EUROPEAN PATENT APPLICATION

(11) **EP 2 677 040 A1**
(43) Date of publication of application: **25.12.2013**
(21) Application number: 13169207.1
(22) Date of filing: 24.05.2013
(51) Int. Cl.: C12Q 1/68

(54) **Probe, chip, kit and method for detection of mycobacterium tuberculosis, non-tuberculous mycobacteria and drug resistant of mycobacterium tuberculosis**

(30) Priority: 22.06.2012 TW 101122512
(71) Applicant: Taichung Veterans General Hospital, Taichung 40705 (TW)
(72) Inventor: Shen, Gwan-Han, 407 Taichung City (TW); Chang, Tzu-Ting, 40705 Taichung (TW)
(74) Representative: Becker Kurig Straus

(57) **Abstract**

This invention provides probes, chip, kit and method for detection the species of *Mycobacterium tuberculosis* (MTB), non-tuberculosis mycobacteria (NTM) and drug resistant of *Mycobacterium tuberculosis.* The purpose of this present invention is archived by hybridization reaction of said probes being selected from the group consisting of SEQ ID NO: 3~23 and 26~36. Efficient and one-step detection for determining the species of NTM, MTB and drug resistance of MTB is achieved via hybridization of probes with specific DNA fragments in MTB, NTM and MTB acquiring drug resistance potency.

SEQUENCE LISTING

<110>  Taichung Veterans General Hospital

<120>  PROBE, CHIP, KIT AND METHOD FOR DETECTION OF MYCOBACTERIUM
TUBERCULOSIS, NON-TUBERCULOUS MYCOBACTERIA AND DRUG RESISTANT OF
MYCOBACTERIUM TUBERCULOSIS

<130>  ARM02PI023EP

<160>  36

<170>  PatentIn version 3.5

<210>  1
<211>  21
<212>  DNA
<213>  Artificial Sequence

<220>
<223>  primer

<400>  1
acctcctttc taaggagcac c                                                21

<210>  2
<211>  21
<212>  DNA
<213>  Artificial Sequence

<220>
<223>  primer

<400>  2
gatgctcgca accactatyc a                                                21

<210>  3
<211>  21
<212>  DNA
<213>  Artificial Sequence

<220>
<223>  probe

<400>  3
tgcatgacaa caaagttggc c                                                21

<210>  4
<211>  20
<212>  DNA
<213>  Artificial Sequence

<220>
<223>  probe

<400>  4
gtggtggggt gtggtstttg                                                  20

<210>  5

<211>  20
<212>  DNA
<213>  Artificial Sequence

<220>
<223>  probe

<400>  5
gtggtggggt gtggactttg                                                    20


<210>  6
<211>  27
<212>  DNA
<213>  Artificial Sequence

<220>
<223>  probe

<400>  6
gggaacataa agtaggcatc tgtagtg                                            27


<210>  7
<211>  21
<212>  DNA
<213>  Artificial Sequence

<220>
<223>  probe

<400>  7
tgcaggccgt gtggagttct c                                                  21


<210>  8
<211>  19
<212>  DNA
<213>  Artificial Sequence

<220>
<223>  probe

<400>  8
cactcggtcc gtccgtgtg                                                     19


<210>  9
<211>  31
<212>  DNA
<213>  Artificial Sequence

<220>
<223>  probe

<400>  9
ggaacataaa gcgagtttct gtagtggtta c                                       31


<210>  10
<211>  20
<212>  DNA
<213>  Artificial Sequence

<220>
<223>  probe

<400>  10
tttgcggtga tgggactgcc                                                   20


<210>  11
<211>  20
<212>  DNA
<213>  Artificial Sequence

<220>
<223>  probe

<400>  11
agcgcgggtg atggaactgc                                                   20


<210>  12
<211>  20
<212>  DNA
<213>  Artificial Sequence

<220>
<223>  probe

<400>  12
ggcaacaccc tcgggtgctg                                                   20


<210>  13
<211>  20
<212>  DNA
<213>  Artificial Sequence

<220>
<223>  probe

<400>  13
cactcggtcg atccgtgtgg                                                   20


<210>  14
<211>  17
<212>  DNA
<213>  Artificial Sequence

<220>
<223>  probe

<400>  14
actcggtcag tccgtgt                                                      17


<210>  15
<211>  20
<212>  DNA
<213>  Artificial Sequence

<220>
<223>  probe

<400>  15
tcggacttgt ctgggtcgtt                                                   20


<210>  16
<211>  20
<212>  DNA
<213>  Artificial Sequence

<220>
<223>  probe

<400>  16
tcgggctctg ttcgagagtt                                                   20


<210>  17
<211>  20
<212>  DNA
<213>  Artificial Sequence

<220>
<223>  probe

<400>  17
acaacaggca atcgccagac                                                   20


<210>  18
<211>  20
<212>  DNA
<213>  Artificial Sequence

<220>
<223>  probe

<400>  18
aacactcggc cagtccgcgt                                                   20


<210>  19
<211>  20
<212>  DNA
<213>  Artificial Sequence

<220>
<223>  probe

<400>  19
aacatctctg ttggtttcgg                                                   20


<210>  20
<211>  21
<212>  DNA
<213>  Artificial Sequence

<220>
<223>  probe

<400>  20
actcggctcg ttctgagtgg t                                                 21

<210>  21
<211>  21
<212>  DNA
<213>  Artificial Sequence

<220>
<223>  probe

<400>  21
aacaacaagc gagaagccga g                                                21


<210>  22
<211>  21
<212>  DNA
<213>  Artificial Sequence

<220>
<223>  probe

<400>  22
aggcttggcc agagctgttg t                                                21


<210>  23
<211>  21
<212>  DNA
<213>  Artificial Sequence

<220>
<223>  probe

<400>  23
tgttgggcag caggcagtaa c                                                21


<210>  24
<211>  19
<212>  DNA
<213>  Artificial Sequence

<220>
<223>  primer

<400>  24
gcgatcacac cgcagacgt                                                   19


<210>  25
<211>  20
<212>  DNA
<213>  Artificial Sequence

<220>
<223>  primer

<400>  25
gcacgtcgcg gacctccagc                                                  20


<210>  26

<211>  18
<212>  DNA
<213>  Artificial Sequence

<220>
<223>  probe

<400>  26
ccagctgagc caattcat                                                    18


<210>  27
<211>  19
<212>  DNA
<213>  Artificial Sequence

<220>
<223>  probe

<400>  27
caattcatgg accagaaca                                                   19


<210>  28
<211>  20
<212>  DNA
<213>  Artificial Sequence

<220>
<223>  probe

<400>  28
ccaattcatg ttccagaaca                                                  20


<210>  29
<211>  18
<212>  DNA
<213>  Artificial Sequence

<220>
<223>  probe

<400>  29
ccgctgtcgg ggttgacc                                                    18


<210>  30
<211>  18
<212>  DNA
<213>  Artificial Sequence

<220>
<223>  probe

<400>  30
ggttgaccca caagcgcc                                                    18


<210>  31
<211>  16
<212>  DNA
<213>  Artificial Sequence

<220>
<223>  probe

<400>  31
gttgacccgc aagcgc                                                        16


<210>  32
<211>  18
<212>  DNA
<213>  Artificial Sequence

<220>
<223>  probe

<400>  32
gggttgacct acaagcgc                                                      18


<210>  33
<211>  17
<212>  DNA
<213>  Artificial Sequence

<220>
<223>  probe

<400>  33
ggttgaccga caagcgc                                                       17


<210>  34
<211>  18
<212>  DNA
<213>  Artificial Sequence

<220>
<223>  probe

<400>  34
gccgactgtc ggcgctgg                                                      18


<210>  35
<211>  16
<212>  DNA
<213>  Artificial Sequence

<220>
<223>  probe

<400>  35
ccgactgttg gcgctg                                                        16


<210>  36
<211>  16
<212>  DNA
<213>  Artificial Sequence

<220>
<223>  probe

<400> 36
ccgactgtgg gcgctg 16

## Description

### BACKGROUND OF THE INVENTION

### 1. Field of the Invention

This invention relates to the field of mycobacteria detection, specially relates to probe, chip, kit and method for detection of mycobacterial nucleic acid in biological sample.

### 2. Description of the Related Art

*Mycobacterium* genus includes *Mycobacterium tuberculosis* complex (MTB) and non-tuberculous mycobacteria (NTM). MTB includes *M tuberculosis, M. africanum, M. bovis* etc. and the other species in *Mycobacterium* genus are all categorized in NTM. So far, there are more than one hundred of identified species of NTM.

MTB is a major pathogen for human pulmonary tuberculosis, which is a epidemic disease causing the most infected and dead patients in the world. In Taiwan, pulmonary tuberculosis is one of legal infectious diseases. Once a diagnosed patient with pulmonary tuberculosis or a MTB infected patient, it has to be notified to the Department of Health and treated with antibiotics.

In the treatment of pulmonary tuberculosis, gradually increased ratio of MTB with drug resistance elevated difficulty in curing, either in Taiwan or worldwide, according statistic reports from World Health Organization and Center of Disease Control in Taiwan. Especially, patients having multidrug resistant tuberculosis (MDR-TB) and extensively drug resistant tuberculosis (XDR-TB) require cautious drug delivering approach.

NTM is a low pathogenic bacteria to human and typically present in the environment. They usually infect human through environmental water, such as condensate water from air conditioner or medical instruments such as ventilator. In clinical, NTM infected patients need not to be announced to the Department of Health. Clinicans have to identify the species of infectious NTM for chosen of appropriated treatment with suitable antibiotics.

Taken together, diagnosis and identification of *mycobacterium* species and drug resistance are important for clinical management. Therefore, it is helpful to rapidly determine whether MTB drug resistance or identify *mycobacterium* species for clinicans trying to select an adequate regiment for patients.

Traditionally, species determination of MTB or NTM in sample isolated from respiratory specimen, mainly sputum, in hospital is achieved by biochemical method which spends 1∼2 months with complex processes. Recently, large-scale hospitals identify MTB and NTM in samples by using BACTEC MGIT 960 system and BD ProbeTec™ ET *Mycobacterium tuberculosis* Complex (CTB) Culture Identification Reagent Pack (Becton Dickinson) within 1∼2 weeks. However, bacterial culture and biochemical identifying method are required to identify the species of NTM. Conventional biochemical methods take times and also frequently interfered by artificial interpretation. In addition, the cost in instruments and reagents for automatic detection, bacterial culture and analysis are too expensive to generally apply in all hospitals.

In previous study, Telenti *et al.* had established database of NTM restriction map by polymerase chain reaction restriction fragment length polymorphism (PCR-RFLP), which is also known as fragment restriction enzyme analysis (PRA), of NTM *hsp65* gene, 65-kilodalton heat shock protein. Species of unknown mycobacterium can be identified by comparison of PRA result from unknown mycobacterium with this database. (Telenti A, Marchesi F, Balz M, Bally F, Bottger EC, Bodmer T. 1993. Rapid identification of mycobacteria to the species level by polymerase chain reaction and restriction enzyme analysis. J Clin Microbiol 31(2): 175-178.)

Kim *et al.* had analyzed *rpo*B gene, polymerase β-subunit-encoding, with PRA (Kim BJ, Lee KH, Park BN, Kim SJ, Bai GH, Kim SJ, Kook YH. 2001. Differentiation of mycobacterial species by PCR-restriction analysis of DNA (342 base pairs) of the RNA polymerase gene (rpoB). J Clin Microbiol. 39:2102-2109; Kim BJ, Hong SK, Lee KH, Yun YJ, Kim EC, Park YG, Bai GH, Kook YH. 2004. Differential identification of Mycobacterium tuberculosis complex and nontuberculous mycobacteria by duplex PCR assay using the RNA polymerase gene (rpoB). J Clin Microbiol 42(3):1308-12.); and then, cross-reference with growth rate, phenotypic characters and two biochemical tests including Tween 80 hydrolysis test and nitrate reduction test to precisely determine the species of mycobacterium (G-H. Shen, C-H. Hung, K-M. Wu, C-H. Chen, C-F. Lin, Y-W. Sun J-H. Chen. Combining the Capilia TB assay with smear morphology for the identifi cation of Mycobacterium tuberculosis complex. Int. J. Tuberc. Lung Dis. 2009; 13(3):371-376). However, practical experiences revealed that the erroneous interpretation occurred in determination of the species of NTM resulted from similar size of PCR-RFLP fragments.

16S-23S rRNA internal transcribed spacer region (ITS) is a transcription region between loci of 16S rRNA and 23S rRNA. There are conserved regions at 5'-end and 3'-end and variant region at central which could be utilized as identification targets for determination of mycobacterium species.

Rifampicin (RIF) could terminate the elongation of translation through binding to the β-subunit of bacterial RNA polymerase encoded from *rpo*B gene. Previous reports have suggested that the MTB stains exhibiting drug resistance to RIF usually contain mutations in *rpo*B gene. Therefore, *rpo*B gene could be utilized as targets to determine the presence of drug resistance in MTB.

Taiwan Pub. No. 201002825, China Pub. No. 101413031 and China Pub. No. 100368559 disclosed detection techniques using PCR with specific primer sets or probes to identify the species of MTB or NTM; however, these techniques comprise many steps and distinguish MTB and NTM roughly without to identify the species of NTM. U.S. Pat. No. 6,025,132 disclosed specific multiple nucleotide probes to identify the specific mycobacterium species exhibiting infectious and pathogenic potential. However, this invention is unable to identify drug resistance of MTB. U.S. Pat. No. 6,329,138, 6,632,607 and 7,252,936 also disclosed specific nucleotide probes to determine the sensitivity and resistance of MTB with RIF; however, these inventions are unable to detect the species of NTM at the same time.

### SUMMARY OF THE INVENTION

In order to improve said problem, this present invention discloses probe, chip, kit and method for detection the species of *Mycobacterium tuberculosis,* non-tuberculosis mycobacteria and drug resistant of *Mycobacterium tuberculosis* to achieve the goal for distinguishing MTB or NTM, identifying the species of NTM, and detecting the drug resistance of MTB by one step manipulation.

In order to achieve the goal, this present invention provides a composition for detection of *Mycobacterium tuberculosis,* non-tuberculosis mycobacteria and drug resistant of *Mycobacterium tuberculosis,* comprising a plurality of probes being selected from the group consisting of: SEQ ID NO: 3∼23, 26∼36.

Wherein, said non-tuberculosis mycobacteria is selected from the group consisting of: *M. abscessus, M. asiaticum, M. avium, M. chelonae, M. fortuitum, M. gordonae, M. intracellulare, M kansasii, M lentiflavum, M malmoense, M marinum, M. scrofulaceum, M. shimodei, M. szulgai* and *M. xenopi.*

In order to achieve the purpose, this present invention provides a chip for detection of *Mycobacterium tuberculosis,* non-tuberculosis mycobacteria and drug resistant of *Mycobacterium tuberculosis,* comprising a solid matrix; and a plurality of probes setting on said matrix, and said probes being selected from the group consisting of: SEQ ID NO: 3∼23, 26∼36.

Wherein, said non-tuberculosis mycobacteria is selected from the group consisting of: *M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai* and *M. xenopi.*

To achieve the purpose, this present invention provides a kit for detection of *Mycobacterium tuberculosis,* non-tuberculosis mycobacteria and drug resistant of *Mycobacterium tuberculosis,* comprising a plurality of probes being selected from the group consisting of: SEQ ID NO: 3∼23, 26∼36.

Wherein, said non-tuberculosis mycobacteria is selected from the group consisting of: *M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai* and *M. xenopi.*

According to a perferred embodiment of said kit, further comprises a solid matrix for setting said probes, wherein said matrix is polystyrene.

According to a perferred embodiment of said kit, further comprises a primer set SEQ ID NO:1 and 2.

According to a perferred embodiment of said kit, further comprises a primer set SEQ ID NO: 24 and 25.

According to a perferred embodiment of said kit, further comprises a hybridization buffer for appropriate environment to perform hybridization reaction of said probes.

According to a perferred embodiment of said kit, further comprises a wash buffer to remove residues which do not hybridize with said probes.

According to a perferred embodiment of said kit, further comprises a streptavidin conjugate alkaline phosphatase to label hybridization products.

According to a perferred embodiment of said kit, further comprises a dilution buffer for said streptavidin conjugate alkaline phosphatase to avoid non-specific binding with said hybridization products.

According to a perferred embodiment of said kit, further comprises a detection reagent to present color via interacting with said streptavidin conjugate alkaline phosphatase.

According to a perferred embodiment of said kit, further comprises a detection buffer to provide required environment for the reaction of color development.

In order to achieve the purposes, this present invention further provides a method for detection of *Mycobacterium tuberculosis,* non-tuberculosis mycobacteria and drug resistant of *Mycobacterium tuberculosis,* comprising following steps: (a) amplifying 16S-23S rRNA ITS gene and *rpo*B gene from sample; (b) providing a plurality of probes to hybridize product from step (a), wherein said probes being selected from the group consisting of: SEQ ID NO: 3∼23, 26∼36; and (c) measuring result of hybridization reaction.

Wherein, said non-tuberculosis mycobacteria is selected from the group consisting of: *M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai* and *M. xenopi.*

According to a perferred embodiment of said method, wherein two primer sets SEQ ID NO: 1 and 2, 24 and 25 were used for amplification in step (a).

According to a perferred embodiment of said method, further comprises following steps: (d) spotting said probes on a solid matrix.

According to these features, this present invention integrates specific probes designed for specific sequence to distinguish MTB versus NTM and the species of NTM. Furthermore, this present invention also provides specific probe for the mutation of drug-resistant MTB. Therefore, this present invention provides a detection technique to determine the species and drug resistance of MTB and NTM in one assay.

The details about structure, features, assembling or utilizing method of this present invention will further explain in following text. However, above-mentioned specification is only for detailed description with the embodiment of this present invention and shall not be construed as a scope limitation of this present invention

### BRIEF DESCRIPTION OF THE DRAWINGS

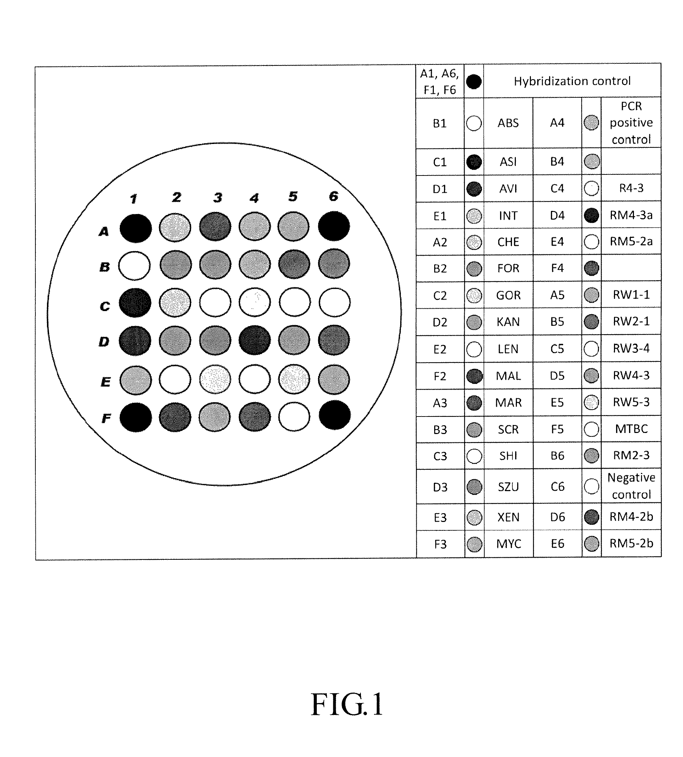

FIG 1**.** The illustration of probes which are used for determining the mycobacterium species and MDR-TB.

A1, A6, F1 and F6 were positive control spots for hybridization to make sure whether all steps of hybridization reactions were archived. A4 was positive control spot which was utilized for confirming the successful reaction of PCR reaction. C6 was negative control spot to detect the contamination of reagents in different steps.

B1 was ABS position spotted with ABS probe to detect *M. abscessus.* C1 was ASI position spotted with ASI probe to detect *M. asiaticum.* D1 was AVI position spotted with AVI probe to detect *M. avium spp. avium.* E I was INT position spotted with INT4 and INT5 probes to detect *M. intracellulare.*

A2 was CHE position spotted with CHE4 probe to detect M. chelonae. B2 was FOR position spotted with FOR2 and FOR7 probes to detect *M. fortuitum.* C2 was GOR position spotted with GOR probe to detect *M. gordonae.* D2 was KAN position spotted with KAN1-1 and KAN4 probes to detect *M. kansasii.* E2 was LEN position spotted with LEN2 probe to detect *M. lentiflavum.* F2 was MAL position spotted with MAL probe to detect *M. malmoense.*

A3 was MAR position spotted with MAR probe to detect *M. marinum.* B3 was SCR position spotted with SCR probe to detect *M. scrofulaceum.* C3 was SHI position spotted with SHI2 probe to detect *M. shimodei.* D3 was SZU position spotted with SZU probe to detect *M. szulgai.* E3 was XEN position spotted with XEN probe to detect *M. xenopi.* F3 was MYC position spotted with MYC-a and MYC-c probes to detect *Mycobacterium* genus.

F5 was MTBC position spotted with MTBC probe to detect *M tuberculosis* complex. A5, B5, C5, D5 and E5 were position on chip to detect wild type *M. tuberculosis* complex. These indicated positions were spotted with probes RW1-1, RW2-1, RW3-4, RW4-3 and RW5-3, respectively.

C4, D4, E4, B6, D6 and E6 were position on chip to detect the drug resistant strains of *M tuberculosis* complex with mutations in *rpoB* gene. These indicated positions were spotted with probes RW4-3, RW4-3a, RW5-2a, RW2-3, RW4-2b and RM5-2b, respectively.

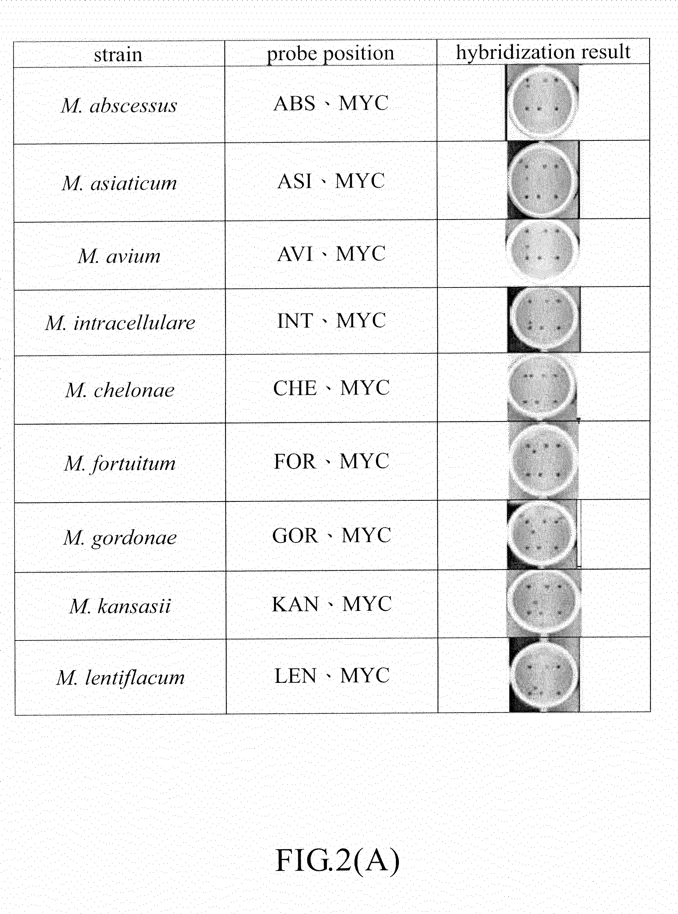

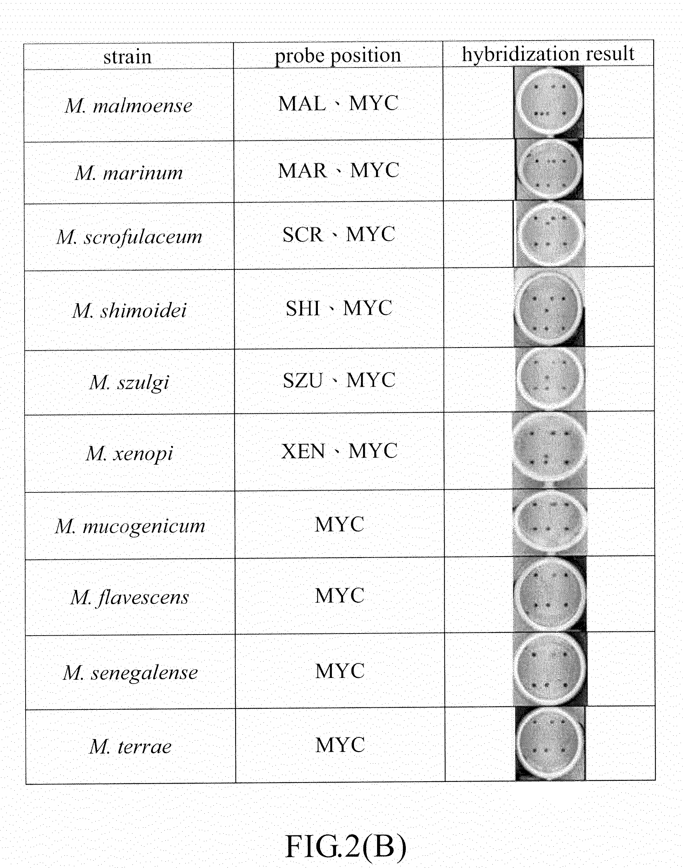

FIG. 2. (A) Hybridization reactions of the common probes for MTB and the specific probes for following species: *M. abscessus, M. asiaticum, M. avium, M. intracellulare, M. chelonae, M. fortuitum, M. gordonae, M. kansasii* and *M. lentiflavum.*

FIG 2. (B) Hybridization reactions of the common probes for mycobacterium genus and the specific probes for following species: *M. malmoense, M. marinum, M. scrofulaceum, M. shimoidei, M. szulgai, M. xenopi, M. mucogenicum, M. flavescens, M. senegalense* and *M. terrae.*

FIG. 3. Hybridization reactions of the common probes for mycobacterium genus, the specific probes for *M tuberculosis* complex and MDR-TB.

FIG. 4.(A) Hybridization reactions of the common probes for mycobacterium genus and various specific probes including a probe for mutant *rpo*B gene of M. *tuberculosis* complex with mutations on codon 511 and codon 526, a probe for mutant rpoB gene of *M. tuberculosis* complex with mutation on codon 516, and a probe for mutant *rpo*B gene of *M. tuberculosis* complex with mutation on codon 526.

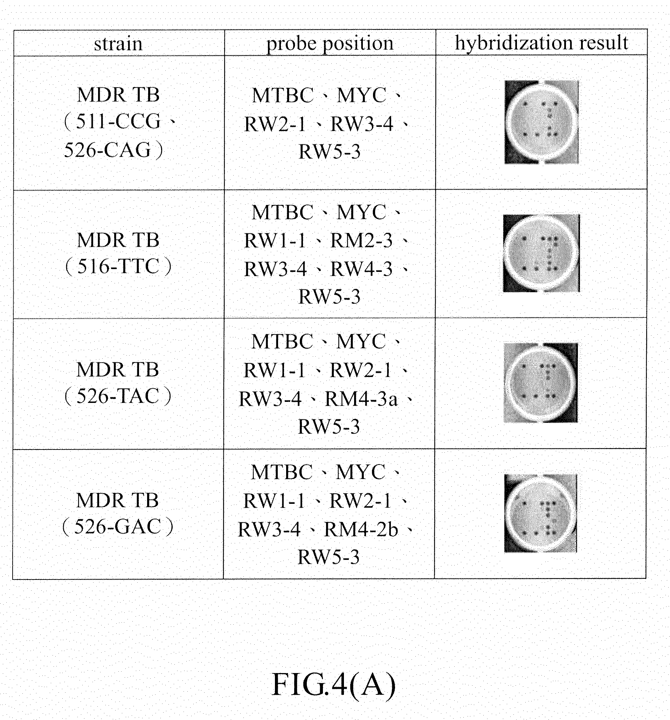

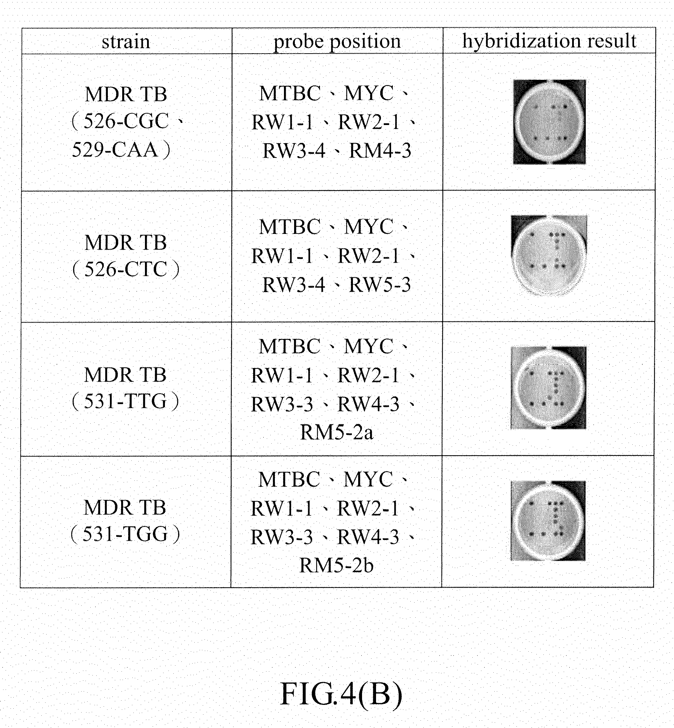

FIG 4.(B) Hybridization reactions of the common probes for mycobacterium genus and various specific probes including a probe for mutant *rpo*B gene of *M. tuberculosis* complex with mutation on codon 526 and codon 529, and a probe for mutant *rpo*B gene of *M. tuberculosis* complex with mutation on codon 531.

### DETAILED DESCRIPTION OF THE INVENTION

The features of this present invention are described in the following examples and figures.

### Example 1: Collection of mycobacteria strains

Reference mycobacteria strains were purchased from American Type Culture Collection (ATCC). MTB strain and comparative strain names were showed in Table 1. Clinical strains were collected from Taichung Veterans General Hospital (Taichung, Taiwan), Chung-Shan Hospital (Taichung, Taiwan), Changhua Christian Hospital (Changhua, Taiwan) and Reference-lab (Taichung, Taiwan).

Mycobacteria strains, wherein clinical samples from the respiratory tract of patient in these indicated hospitals or lab, identified by traditional biochemical and molecular methods include 9 *M tuberculosis* complex, 9 *M. abscessus,* 1 *M. asiaticum,* 3 *M. avium,* 3 *M. chelonae,* 9 *M. fortuitum,* 9 *M. gordonae,* 9 *M. intracellulare,* 9 *M. kansasii,* 2 *M. lentiflavum,* 1 *M. malmoense,* 2 *M. marinum,* 1 *M. scrofulaceum,* 2 *M shimodei,* 1 *M. szulgai* and 2 *M*. *xenopi.* The strains and species of those strains were shown in Table 1.

The identification methods utilized including pre-processes of respiratory tract specimens, culture of bacterial clones, identification of acid-fast bacillus, traditional biochemical methods and molecular biological methods were briefly described as below.

### 1. Pre-processes of the samples from respiratory tract

Samples collected from the respiratory tract of patients were digested by NALC (N-acety-L-cysyeine)-2% NaOH decontamination procedure, centrifugal concentration, and sputum dissolving agents first. The processed specimens were culture on Löwenstein-Jensen medium (hereafter "L-J medium") or Middlebrook 7H11 medium (hereafter "7H11 medium") in BD BACTEC 960 MGIT tubes (mycobacterial growth indicator tube, hereafter "MGIT tube").

### 2. Culture of bacterial strains

L-J medium and 7H11 medium were incubated at 37 °C, 5% CO₂ microbiological incubator. After scanning labels of MGIT tubes by BACTEC MGIT 960 system, MGIT tubes were incubated in incubator. The culture period of BD BACTEC MGIT 960 system is 42 days. Generally, positive signal indicating growth of mycobacterium in medium would presence within 7∼10 days while mycobacterium exists in samples.

### 3. Identification of acid-fast bacillus

Acid-fast stain was performed to confirm the existence of mycobacteria when MGIT instrument detected positive signal for the growth of mycobacteria. Morphology of acid-fast bacillus was recorded when the existence of mycobacterium in MGIT tube was present by positive staining of acid-fast stain. Growth of colony in L-J medium was also compared with the bacterial growth in MGIT tube. Consistent result of colony formation on L-J medium would be utilized to assist the result of bacterial growth in MGIT tube.

### 3.1 CTB analysis

CTB analysis is utilized a detection kit, BD ProbeTec™ ET *Mycobacterium tuberculosis* complex (CTB) Culture Identification Reagent Pack, developed by Becton Dickinson company for detecting the presence of MTB. In CTB analysis, the existence of MTB was detected by strand displacement amplification (SDA) which amplifies specific repeated sequence IS6110 in MTB and labels amplified DNA fragment with luminescence for detection. The growth of MTB in MGIT tube was detected by manufacturer's instruction. Positive signal from this assay suggests that existing DNA of MTB in sample tested.

### 3.2 Biochemical assays

Traditional biochemical assay will be performed to confirm the species of mycobacterium when there were more than two specimens detected from the colony in MGIT tube collected from the respiratory specimens of patients. Biochemical assays including Arysulfatase assay, Catalase assay, Tolerance to 5% NaCl assay, Niacin accumulation test, Tween 80 hydrolysis assay, urease assay were performed following the mycobacterium laboratory handbook published from Centers for Disease Control, R.O.C., Taiwan, in 2004.

### 3.3 PCR-RFLP identification

Chromosomal DNA extracted from each bacterial colonies were subjected for PCR to amplify *hsp*65 gene for restriction enzyme digestion by BstEII and HaeIII. The comparison between the restriction enzyme digestion map with the *hsp*65 RFLP pattern in the database, which established by Telenti et al., was performed to identify the MTB species (http://app.chuv.ch/prasite/index.html).

### Experiment 2: PCR amplification of 16S-23S rRNA ITS gene in MTB

Amplification of the region in 16S-23S rRNA ITS locus by PCR reaction was performed with the primer sets Sp1 (SEQ ID NO: 1) and Sp2 (SEQ ID NO: 2) to amplify the common region for all mycobacteria species (Xiong L, Kong F, Yang Y, Cheng J, Gilbert GL. 2006. Use of PCR and reverse line blot hybridization macroarray based on 16S-23S rRNA gene internal transcribed spacer sequences for rapid identification of 34 mycobacterium species. J Clin Microbiol 44(10):3544-50).

The single colony of NTM was sampled and re-suspended in 40µL ddH₂O which was further heated for 10 minutes to obtain DNA containing supernatant. In addition, DNA of MTB was prepared by *M tuberculosis* Complex (CTB) Culture Identification Reagent Pack purification kit, BD ProbeTec™ ET, for the further PCR reaction. Total volume of PCR reaction was up to 50µL which was sequentially added with 28.75µL of ddH₂O, 5µL of DNA template, 1µL 10µM Sp1 primer (SEQ ID NO:1), 1µL of 10µM Sp4 (SEQ ID NO:2, wherein Y means T or C), 4µL of 2.5mM dNTP, 10µL of 5x buffer (Promega) and 0.25µL GO Taq polymerase (5000 units/mL, Promega). Following PCR reaction, gel electrophoresis was performed to detect PCR products which might be 212-300 base pairs dependent on different species of the bacterial colonies (Roth A, Reischl U, Streubel A, Naumann L, Kroppenstedt RM, Habic Fischer M, Mauch H. 2000. Novel diagnostic algorithm for identification of mycobacteriausing genus-specific amplification of the 16S-23S rRNA gene spacer and restriction endonucleases. J. Clin Microbiol 38(3):1094-1104.).

In this experiment, DNA extracted from 16 reference mycobacteria bacterial strain and clinical strains were subjected for PCR reaction. Species, number of strains and size of PCR product were shown in Table 1. below.

**Table 1. The PCR product of ITS gene in the MTB standard clone and clinical clone.**

| Species | Analyzed Clone | | Size of PCR product |
|---|---|---|---|
| | Clinical clone | Standard clone | |
| *M. tuberculosis* complex | O151 · P36 · Q136 · R54 · R195 · R205 · S63 · S10 · S12 | H37Rv | 221 |
| *M. abscessus* | 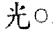 · 4871 · type II S19 · IS 1-2 · type I S21 · 95 CAP-9 · 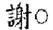 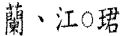 · IS 3-2 | ATCC 19977 | 257 |
| *M. asiaticum* | 96-CAP-5 | ATCC 25276 | 220 |
| *M. avium* | cch7 · cch10 · V213 | ATCC 25291 | 219 |
| *M. chelonae* | ch 39 · V200 · 95-7 | ATCC 35752 | 257 |
| *M. fortuitum* | V61 | - | 257 |
| | V9 · V13 · V65 | - | 280 · 300 |
| | V7 · V12 · V16 · V23 | - | 257 · 300 |
| | V205 | - | 280 |
| | - | ATCC 6841 | 257 · 280 |
| *M. gordonae* | V43 · V45 · V121 · V132 · V138 · V142 · V147 · V155 · V167 | ATCC 14470 | 211 |
| *M. intracellulare* | V17 · V19 · V193 · V194 · V195 · cch11 · cch12 · cch21 · cch37 | ATCC 13950 | 219 |
| *M. kansasii* | V128 · V168 · V169 · V179 · V182 · V185 · cch1 · cch14 · cch23 | ATCC 12478 | 220 |
| *M. lentiflavum* | cch24 · cch30 | - | 225 |
| *M. malmoense* | cch13 | ATCC 29571 | 219 |
| *M. marinum* | V74 · 97-CAP-1 | ATCC 927 | 220 |
| *M. scrofulaceum* | 96-CAP-2 | ATCC 19981 | 220 |
| *M. shimodei* | V219 · V229 | ATCC 27962 | 230 |
| *M. szulgai* | cch34 | ATCC 29716 | 220 |
| *M. xenopi* | 95-CAP-1 · 97-CAP-11 | ATCC 19250 | 205 |

### Experiment 3: Sequencing analysis of 16S-23S rRNA ITS gene of mycobacteria

Since gel electrophoresis showed two PCR products amplified from 16S-23S rRNA ITS of *M. fortuitum* with similar size. Therefore, these bands were further eluted and cloned by yT&A cloning for DNA sequencing. First, the bands containing gel were purified by QIA quick Gel Extraction kit (QIAGEN, Germany) to prepare the inset DNA. Following, ligation was performed with 3:1 molar ratio of insert DNA to vector DNA for the transformation.

The PCR products amplified from 16S-23S rRNA ITS gene of one colony of *M. asiaticum, M. malmoense, M. scrofulaceum* and *M. szulgai;* three colonies of *M. avium* and *M. chelonae;* two colonies of *M. lentiflavum, M. marinum, M. shimodei* and *M. xenopi;* and four colonies of other strains among these 16 clinical strains were sequenced by Tri-i biotech, Inc. (Taiwan). The primer sets used for the DNA sequencing is Sp1 (SEQ ID NO: 1). The PCR product of 16S-23S rRNA ITS gene from *M. fortuitum* was cloned by yT&A cloning for DNA sequencing with T7 primer.

### Experiment 4: Designing specific probes for identifying mycobacteria

The acquired sequences were further analyzed by DNAMAN (version 4.11). Comparison analysis was performed to find out sequences of specific probes and common probes among these 16 mycobacteria strains. Poly (T) containing 15 T at the 5'-end was followed by sequences of specific probes for each mycobacteria strains. Probes and corresponding mycobacteria strains were shown in the Table 2. Wherein, identification of *Mycobacterium* genus, *M. fortuitum, M. intracellulare* and *M. kansasii* required two independent probes, and the identification of other mycobacteria strains required one probe.

**Table 2. The sequence of common and specific probes for MTB**

| Identified species | Probe | Sequence (5'→ 3') | SEQ ID NO. |
|---|---|---|---|
| *M. tuberculosis* complex | MTBC | TGCATGACAACAAAGTTGGCC | 3 |
| *Mycobacterium* genus | MYC-c | GTGGTGGGGTGTGGTSTTTG | 4 |
| *Mycobacterium* genus | MYC-a | GTGGTGGGGTGTGGACTTTG | 5 |
| *M. abscessus* | ABS | GGGAACATAAAGTAGGCATCTGTAG TG | 6 |
| *M. asiaticum* | ASI | TGCAGGCCGTGTGGAGTTCTC | 7 |
| *M. avium* | AVI2 | CACTCGGTCCGTCCGTGTG | 8 |
| *M. chelonae* | CHE4 | GGAACATAAAGCGAGTTTCTGTAGT GGTTAC | 9 |
| *M. fortuitum* | FOR2 | TTTGCGGTGATGGGACTGCC | 10 |
| | FOR7 | AGCGCGGGTGATGGAACTGC | 11 |
| *M. gordonae* | GOR | GGCAACACCCTCGGGTGCTG | 12 |
| *M. intracellulare* | INT4 | CACTCGGTCGATCCGTGTGG | 13 |
| | INT5 | ACTCGGTCAGTCCGTGT | 14 |
| *M. kansasii* | KAN1-1 | TCGGACTTGTCTGGGTCGTT | 15 |
| | KAN4 | TCGGGCTCTGTTCGAGAGTT | 16 |
| *M. lentiflavum* | LEN2 | ACAACAGGCAATCGCCAGAC | 17 |
| *M. malmoense* | MAL | AACACTCGGCCAGTCCGCGT | 18 |
| *M. marinum* | MAR | AACATCTCTGTTGGTTTCGG | 19 |
| *M. scrofulaceum* | SCR | ACTCGGCTCGTTCTGAGTGGT | 20 |
| *M. shimodei* | SHI2 | AACAACAAGCGAGAAGCCGAG | 21 |
| *M. szulgai* | SZU | AGGCTTGGCCAGAGCTGTTGT | 22 |
| *M. xenopi* | XEN | TGTTGGGCAGCAGGCAGTAAC | 23 |

### Experiment 5: Collection of MTB exhibiting drug resistance

Reference strain of MTB (H37Rv) was purchased from ATCC and clinical strains of MTB exhibiting drug resistance were collected by Reference-lab. Those clinical strains exhibiting tested and confirmed drug resistance were showed in Table 3. Preparation of samples from respiratory specimens, bacterial culture, acid-fast staining, biochemical assays and molecular biological methods were described in Experiment 1.

Drug sensitivity test was performed while MTB was isolated according indicated methods and processes. The first line drug for the drug sensitivity test was streptomycin (SM), isoniazid (INH), rifampicin (RIF) and ethambutol (EMB). Culture medium plates purchased from Bio Concept (Taiwan, Taipei) were used for the drug sensitivity test of MTB with manufacturer's instruction. The concentration of test drugs in culture medium plates is 0.2 µg/ml and 1.0 µg/ml for INH; 1.0 µg/ml for RIF; 2.0 µg/ml and 10 µg/ml for SM; 5 µg/ml and 10 µg/ml for EMB.

### Experiment 6: PCR amplification of rpob gene in drug resistant strain of MTB

The primer sets, rpoF-1(SEQ ID NO: 24) and rpo8-1(SEQ ID NO: 25), were used to amplify *rpoB* gene. Total volume of PCR reaction was 50µL which was sequentially added with 18µL of ddH₂O, 5µL of DNA template, 1µL 10µM rpoF-1 (SEQ ID NO: 24), 1µL 10µM rpo8-1 (SEQ ID NO: 25), 25µL 2x GO Taq Colorless Master MIX (Promega). PCR conditions are 95°C for 5 minutes; 95°C for 30 seconds, 60°C for 30 seconds, and 72°C for 45 seconds for 30 cycles; 72°C for 5 minutes. After PCR reaction, gel electrophoresis was performed in 2% agarose gel to detect PCR product with predicted size at 196 base-pairs. The results show in Table 3.

**Table 3. PCR amplification of rpoB gene from standard MTB strains and clinical MTB strains with drug resistance**

| Drug resistance | Selected strain | | PCR product (bp) |
|---|---|---|---|
| | Clinical strain | Standard strain | |
| None | - | H37Rv | 196 |
| Rifampicin | MDR TB 1 ∼ 39 · 41 ∼ 49 | - | 196 |

### Experiment 7: Sequences analysis of rpoB gene in MTB with drug resistance

The amplified DNA from 48 colonies of MTB with drug resistance were further sequenced and analyzed by BLAST system on the National Center of Biotechnology Information (NCBI) website (http://blast.ncbi.nlm.nih.gov/Blast.cgi). The results were further compared with the *rpo*B gene sequence of MTB (H37Rv) standard strain to identify the position and sequence of mutations occurred in each drug-resistant MTB strains. The results show in Table 4 below.

**Table 4. Positions and sequence alterations of the mutations in each drug-resistant MTB strains**

| Bacterial strains | Mutation position | Sequence alteration |
|---|---|---|
| MDR TB 41 | 511 | CTG→CCG |
| MDR TB 27 | 513 | CAA→CTA |
| MDR TB 6 · 2 · 34 | 516 | GAC→TTC |
| MDR TB 21 · 45 | 516 | GAC→TAC |
| MDR TB 49 | 522 | TCG→TTG |
| MDR TB 1 · 3 ·19 | 526 | CAC→TAC |
| MDR TB 25 | 526 | CAC→GAC |
| MDR TB 13 | 526 | CAC→CGC |
| | 529 | CGA→CAA |
| MDR TB 39 | 511 | CTG→CCG |
| | 526 | CAC→CAG |
| MDR TB 44 | 526 | CAC→CTC |
| MDR TB 35 | 529 | CGA→CTA |
| MDRTB 2 · 45 · 7 · 8 · 9 · 10 · 12 · 14 · 15 · 16 · 17 · 18 · 22 · 23 · 24 · 26 · 29 · 30 · 31 · 33 · 37 · 38 · 43 · 46 · 47 · 48 | 531 | TCG→TTG |
| MDRTB 11 · 20 · 28 · 42 | 531 | TCG→TGG |
| MDR TB 36 | 533 | CTG→CCG |

The results revealed that the mutations occurred in 48 drug-resistant MTB strains were all point mutation locating at codon 511 to 533. There were 31 strains (31/48) exhibiting mutation on codon 531, 27 stains revealed TCG→TTG mutation and other 4 strains showed were TCG→TGG mutation. 5 strains (5/48) showed mutations on codon 526, 3 strains showed the CAC→TAC mutation, I strain showed CAC→GAC mutation and another one strain showed CAC→CTC mutation. 5 strains (5/48) exhibited mutations on codon 516, 3 strains showed the GAC→TTC dinucleotides mutation and 2 strains showed the GAC→TAC mutation. 1 out of 48 strains showed CTG→CCG mutation on codon 511. 1 strain showed the CAA→CTA mutation on codon 513. 1 strain showed the TCG→TTG mutation on codon 522. 1 strain showed the CGA→CTA mutation on codon 529. 1 strain showed the CTG→ CCG mutation on codon 533. In addition, there were 2 strains contain two mutations on different position. There were one strain showed CAC→CGC mutation on codon 526 and CGA→CAA mutation on codon 529, another strain showed CTG→CCG mutation on codon 511 and CAC→CAG mutation on codon 526.

### Experiment 8. Probe design for drug-resistant MTB

Probes were designed according to the mutation positions and sequences in *rpo*B gene of various drug-resistant MTB strains and synthesized with poly (T) comprising 15 T at the 5'-end. The probes were shown in Table 5 below.

**Table 5. The probe sequences for drug-resistant MTB**

| Probe | Sequence (5'→3') | SEQ ID NO. | Detecting position |
|---|---|---|---|
| RW1-1 | CCAGCTGAGCCAATTCAT | 26 | 510 · 511 · 512 · 513 · 514 |
| RW2-1 | CAATTCATGGACCAGAACA | 27 | 513 · 514 · 515 · 516 · 517 · 518 |
| RM2-3 | CCAATTCATGTTCCAGAACA | 28 | 513 · 514 · 515 · 516 · 517 · 518 |
| RW3-4 | CCGCTGTCGGGGTTGACC | 29 | 520 · 521 · 522 · 523 · 524 · 525 |
| RW4-3 | GGTTGACCCACAAGCGCC | 30 | 524 · 525 · 526 · 527 · 528 |
| RM4-3 | GTTGACCCGCAAGCGC | 31 | 524 · 525 · 526 · 527 · 528 |
| RM4-3a | GGGTTGACCTACAAGCGC | 32 | 523 · 524 · 525 · 526 · 527 · 528 |
| RM4-2b | GGTTGACCGACAAGCGC | 33 | 524 · 525 · 526 · 527 · 528 |
| RW 5-3 | GCCGACTGTCGGCGCTGG | 34 | 529 · 530 · 531 · 532 · 533 |
| RM5-2a | CCGACTGTTGGCGCTG | 35 | 529 · 530 · 531 · 532 · 533 |
| RM5-2b | CCGACTGTGGGCGCTG | 36 | 529 · 530 · 531 *·* 532 · 533 |

RW1-1 probe was designed for the position between codon 510 to 514 of *rpo*B in H37Rv reference MTB strain. RW2-1 probe was designed for the position between codon 513 to 518 of *rpo*B in H37Rv reference MTB strain. In addition, RM2-3 probe was designed for the mutation on codon 516 and covered the region from codon 513 to 518 according to the sequencing result of MDR TB 6, 32 and 34 shown in Table 4.

RW3-4 probe was designed for the position between codon 520 to 525 of *rpo*B in H37Rv reference MTB strain. RW4-3 probe was designed for the position between codon 524 to 528 of *rpo*B in H37Rv reference MTB strain. RM4-3 probe was designed for the mutation on codon 526 and covered the region from codon 524 to codon 528 according to the sequencing result from MDR TB 13 in Table 4. Moreover, RM4-3a probe was designed for the mutation on codon 526 and covered the region from codon 523 to 528 according to the sequencing result from MDR TB 1, 3 and 19 in Table 4. RM4-3b probe was designed for the mutation on codon 526 and covered the region from codon 524 to 528 according the sequencing result from MDR TB 25 in Table 4.

RW5-3 probe was designed for the position between codon 529 to 533 of *rpo*B in H37Rv reference MTB strain. Moreover, RW5-2a probe was designed for codon 529 to 533 to detect mutation on codon 531 according to the sequencing results of MDR TB 2, 4, 5, 7∼10, 12, 14∼18, 22∼24, 26, 29∼31, 33, 37, 38, 43, 43 and 46∼48 in Table 4. In addition, RM5-2b was designed for codon 529 to 533 to detect mutation on codon 531 according to the sequencing results of MDR TB 11, 20, 28 and 42 in Table 4.

### Experiment 9: Duplex PCR reaction for amplifying 16S-23S rRNA and rpoB genes

The primer set Sp1 (SEQ ID NO: 1) and Sp4 (SEQ ID NO: 2) for 16S-23S rRNA ITS and another primer set, rpoF-1 (SEQ ID NO: 24) and rpo8-1 (SEQ ID NO: 25) for *rpo*B genes, were utilized for duplex PCR reaction to amplify 16S-23S rRNA ITS and *rpo*B gene in the same PCR reaction. Notably, the primers including Sp4 (SEQ ID NO: 2) and rpo8-1 (SEQ ID NO: 25) were labeled with biotin for the duplex PCR. Equal amount of two primer sets including rpoF-1 (SEQ ID NO: 24) and rpo8-1 (SEQ ID NO: 25), Sp1 (SEQ ID NO: 1) and Sp4 (SEQ ID NO: 2), were added for the duplex PCR reaction. Total volume of PCR reaction was 25µL which contains DNA template, 0.4 µM of rpoF-1 and rpo8-1, 0.4 µM of Sp1 and Sp4, 0.2mM dNTP, 1.5 mM MgCl2, 1×PCR buffer, 2×10² copies/µL control DNA template and 0.3µL DNA polymerase. The heating condition for duplex PCR reaction is 95°C for 5 minutes; 95 °C for 30 seconds, 60°C for 30 seconds, and 72°C for 1 minute for 35 cycles; 72°C for 10 minutes. Products of duplex PCR reaction should amplify DAN fragments with 212-300 bp (16S-23S rRNA ITS gene) dependent on detected strains. Furthermore, DNA fragment with 196 bp (*rpo*B gene) would be amplified while MTB exists in the detecting sample.

### Experiment 10: Preparation of chip

Preparation of probe containing reagent by dissolving 100 µmole synthesized probes by 1× probe buffer (DR.Chip Biotech, Inc, Miaoli, Taiwan) with concentration at 10µM. Probes were spotted on the destined positions of polystyrene plates by automatic spotting machine (DR. Fast Spot, DR.Chip Biotech, Inc). Positions of the spotted probes on chip were showed in Fig.1. Wherein, there were 36 probe spots including 28 detecting probe spots, 4 positive control spots for hybridization reaction, 1 negative control spot for hybridization reaction and 1 positive control spot for PCR reaction on chip. Spotted chip was treated with UV to fix probes on plate and then washed by ddH₂O and dried. Finally, prepared chip was stored at 4°C.

### Experiment 11: Hybridization reaction

25µL biotinated PCR production from duplex PCR was heat at 95°C for 5 minutes to denature and chilled on ice for 2 minutes. Spotted chip was loaded with 200µL hybridization buffer (DR.Hyb™ buffer, DR.Chip Biotech, Inc) in each well and followed by loading with 5µL of PCR product. Chip was sealed with cellophane to prevent contamination between wells. After that, chip was shacked and incubated in the hybridization oven at 55°C for 40 minutes.

After hybridization reaction, cellophane was removed from chip and discarded the hybridization buffer. Then, wells were wash by adding 200µL wash buffer (DR.Chip Biotech, Inc) and incubating for 3 minutes, thrice. Wash buffer was discard carefully as complete as possible in the last wash. 200µL blocking reagent (DR.Chip Biotech, Inc.) which contains 0.2µL Strep-AP (DR.Chip Biotech, Inc.) was added into each well and incubated for 20 minutes at room-temperature. After the incubation, Strep-AP reagent was discarded and added wash buffer for 3 minutes to wash, thrice. Washing buffer had to be completely discarded in the last wash. 200µL of detection buffer (DR.Chip Biotech, Inc.) was added into wells for rinse and discarded. 200µL of detection buffer which contains 4µL detection reagent (NBT/BCIP, DR.Chip Biotech, Inc.) was added into wells and incubated in dark for 7 minutes. After reaction, detection reagent was discarded from wells which were followed by wash with ddH₂O. Finally, wash ddH₂O was removed for analysis by eye or DR. AiM reader (DR.Chip Biotech, Inc.).

### Experiment 12: Chip analysis for the clinical samples

Among 216 strains from clinical samples for this test, 209 strains were correctly determined, 1 strain was not successfully cultured but diagnosed by chip, 6 stains revealed inconsistent results in bacterial culture and chip analysis. Taken together, overall reaction sensitivity is 97.2% and specificity is 96.8%. In TB test, reaction sensitivity is 100% and specificity is 98.9%. Analyzing results are shown in Table 6 below.

**Table 6. Comparison of chip analysis and bacterial culture**

| chip analysis (strain number) | Culture (strain number) |
|---|---|
| *M. abscessus* (21) | *M. abscessiss*(15) |
| | RGM (2) |
| | NTM (4) |
| *M. avium* (6) | *M avium* (1) |
| | MAC (1) |
| | NTM (4) |
| *M. chelonae* (7) | *M. chelonae* (1) |
| | NTM (6) |
| *M. fortuitum* (9) | *M. fortuitum* (9) |
| *M. gordonae* (9) | *M gordonae* (7) |
| | *M. haemophilum* (1) |
| | NTM (1) |
| *M. intracellulare* (7) | *M. intracellulare* (2) |
| | MAC (1) |
| | NTM (4) |
| *M. kansasii* (11) | *M. kansasii* (8) |
| | NTM (3) |
| *M. lentiflavum* (1) | *M. gordonae* (1) |
| *M. malmoense* (1) | *M. malmoense* (1) |
| *M. marinum* (1) | NTM (1) |
| *M. scrofulaceum* (2) | *M. scrofulaceum* (1) |
| | NTM (1) |
| *M szulgai* (4) | *M. kansasii* (2) |
| | NTM (2) |
| *M. xenopi* (3) | *M. xenopi* (1) |
| | NTM (2) |
| *M tuberculosis* complex (109) | *M tuberculosis* complex (109) |
| *M. tuberculosis* complex & *M. intracellulare* (4) | *M tuberculosis* complex (4) |
| *M. tuberculosis* complex & *M. lentiflavum* (2) | *M tuberculosis* complex & NTM (1) |
| | no growth (1) |
| *M avium & M. chelonae* (1) | NTM (1) |
| *M. abscessus & M. chelonae* (1) | NTM () |
| *M. intracellulare & M. fortuitum* (1) | NTM (1) |
| *M intracellulare & M. szulgai* (1) | NTM (1) |
| *M avium & M kansasii* (1) | *M. kansasii* (1) |
| NTM (12) | *M. fortuitum* (2) |
| | *M. mucogenicum* (1) |
| | *M. peregrinum* (1) |
| | NTM (8) |
| No signal (2) | Tsukamurella (1) |
| | Nocardia (1) |

There were 21 strains determined as *M. abscessus* according to hybridization result in chip assay shown in FIG. 2A. In addition, comparing results from bacterial culture revealed that 15 strains were *M. abscessus,* 2 strains were Rapid Growing Mycobacteria (RGM), and 4 strains were NTM.

6 strains were determined as *M avium* according to hybridization result in chip assay in FIG. 2A. Comparing results from bacterial culture showed that 1 strain was *M. avium,* 1 strain was *Mycobacterium avium* complex (MAC), and 4 strains were NTM.

7 strains were determined as *M. chelonae* according to hybridization result in chip assay shown in FIG. 2A. Comparing results from bacterial culture showed that 1 strain was *M. chelonae,* and 6 strains were NTM.

9 strains were determined as *M. fortuitum* according to hybridization result in chip assay shown in FIG. 2A which was consistent with the bacterial culture assay.

9 strains were determined as *M gordonae* according to hybridization result in chip assay shown in FIG. 2A. Comparing results from bacterial culture showed that 7 strains were *M. gordonae,* 1 strain was *M. haemophilum* and 1 strain was NTM. Result of *M. haemophilum* was inconsistent between hybridization result in chip assay and bacterial culture assay.

7 strains were determined as *M. intracellulare* according to hybridization result in chip assay shown in FIG. 2A. Comparing results from bacterial culture showed that 2 strains *M. intracellulare,* 1 strain was MAC and 4 strains were NTM.

11 strains were determined as *M kansasii* according to hybridization result in chip assay shown in FIG. 2A. Comparing results from bacterial culture showed that 8 strains *M kansasii* and 3 strains were NTM.

1 strain was determined as *M. lentiflavum* according to hybridization result in chip assay shown in FIG. 2A. However, culture assay showed that the testing bacterium was *M. gordonae.* Results of chip analysis and bacterial culture assay were inconsistent.

1 strain was determined as *M. malmoense* according to chip hybridization shown in FIG. 2A. In addition, bacterial culture assay also showed that this strain was *M. malmoense.*

1 strain was determined as *M. marinum* according to chip hybridization shown in FIG. 2A. In addition, bacterial culture assay also showed that this strain was *M. marirtum.*

2 strains were determined as *M. scrofulaceum* according to chip hybridization shown in FIG. 2A. Comparing results from bacterial culture showed that I strain was *M. scrofulaceum* and another one was NTM.

4 strains were determined as *M. szulgai* according to chip hybridization shown in FIG. 2A. Comparing results from culture showed that 2 strains were *M. kansasiiand* and 2 were NTM. Results of chip analysis and bacterial culture assay were inconsistent.

1 strain was determined as *M. xenopi* according to chip hybridization shown in FIG. 2A. In addition, comparing results from bacterial culture showed that 1 strain was *M. xenopi* and 2 strains were NTM.

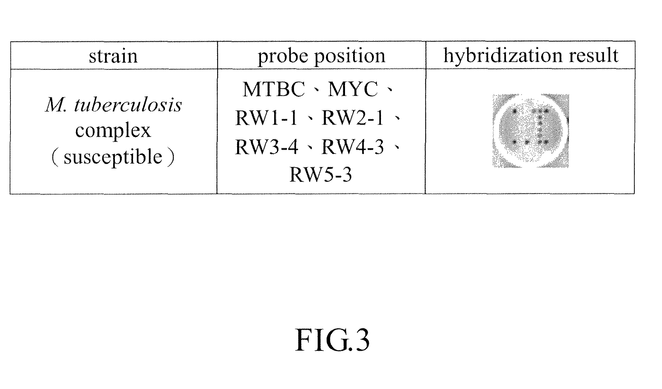

109 strains were determined as *M tuberculosis* complex according to chip analysis shown in FIG. 3B and bacterial culture assay showed consistent result as chip analysis.

4 strains were diagnosed as *M tuberculosis* complex and *M. intracellulare* according to chip analysis. In addition, results of bacterial culture assay showed that 4 strains were *M tuberculosis* complex.

2 strains were diagnosed as *M tuberculosis* complex and *M. lentiflavum* according to chip analysis. Result of culture assay showed that 1 strain was mixed with *M tuberculosis* complex and NTM, and another strain did not grew in bacterial culture assay.

1 strain showed positive signal in chip assay for *M. avium* and *M. chelonae.* In addition, results of bacterial culture assay showed that this strain was NTM.

1 strain showed positive signal in chip assay that determined this strain as *M. abscessus* and *M. chelonae.* Moreover, results of bacterial culture assay showed that this strain was NTM.

1 strain showed positive in chip assay that determine this strain as *M. intracellulare* and *M. fortuitum.* In addition, results of bacterial culture assay showed that this strain was NTM.

1 strain showed positive in chip assay that determine this strain as *M. intracellulare* and *M. szulgai.* In addition, results of bacterial culture assay showed that this strain was NTM.

I strain showed positive in chip assay that determined this strain as *M. avium* and *M kansasii.* In addition, results of bacterial culture assay showed that this strain was *M kansasii.*

12 strains were determined as NTM according to chip analysis. 2 strains were *M. fortuitum,* 1 stain was *M. mucogenicum,* 1 stain was *M. peregrinum* and 8 strains were NTM according to biochemical assays. Result from biochemical assay which indicated this strain as *M. fortuitum* was inconsistent from result of chip analysis.

There were 2 strains revealed negative in chip analysis, 1 strain was Tsukamurella and the another was Nocardia. Both of them belong to bacteria that could not be detected by this chip platform which did not include specific probe for these two bacteria species. Hence, these two strains revealed negative result in chip analysis.

Furthermore, clinical strain *M. mucogenicum,* standard strains including *M. flavescens* (ATCC 14474), *M. senegalense* (ATCC 35796) and *M. terrae* (ATCC 15755) were subjected for chip analysis to obtain hybridization result shown in FIG. 2B. Since the designed chip in this invention did not include specific probes for these indicated bacterial strains. Therefore, chip analysis of these strains just revealed positive hybridization signal from common probe for mycobacterium. This result suggests that even strain wants to be tested not belonged to 16 mycobacterium species in this assay; present chip could still determine whether it belongs to mycobacterium genus.

In addition, RIF drug resistance test performed on 109 strains of *M. tuberculosis* complex in Table 6 by this chip platform. Hybridization results showed in FIG. 4A and 4B. Comparison of chip analysis and drug sensitivity test was showed in Table 7. There were 15 RIF resistant strains determined by chip assay including 14 RIF resistant strains and 1 INH resistant strain examined by drug sensitivity test. Other 94 stains were determined as non-drug resistant MTB according to chip analysis and bacterial culture test.

**Table 7. Comparison of chip analysis and drug sensitivity test of drug sensitive test**

| Chip analysis (strains number) | Drug sensitivity test (strains number) |
|---|---|
| *M tuberculosis* complex (94) | *M. tuberculosis* complex (94) |
| MDR TB (15) | MTB- RIF resistance (14) |
| | MTB- INH resistance (1) |

Detection technique based on molecular biology provides a platform to rapidly determine the species and drug resistance of bacteria to be tested. The chip platform in this invention could determine 16 mycobacterium species and RIF resistance strains. In addition, existence of RIF resistant strains probably exhibiting INH resistance. There will be multiple drug resistance if both RIF and INH resistant to be determined. This information is helpful for clinicians to appropriately modify the management for patient treatment.

The above-mentioned specification is only for detailed description with the examples of this present invention and shall not be construed as a scope limitation of this present invention. Any modification or change without departing from the features of this present invention or any equivalent thereof shall be included in the scope of this present invention defined in the following claims.

## Claims

1. A composition for detection of Mycobacterium tuberculosis, non-tuberculosis mycobacteria and drug resistant of Mycobacterium tuberculosis, being **characterized in that** the composition comprises a plurality of probes being selected from the group consisting of: SEQ ID NO: 3∼23, 26∼36.

2. The composition according to claim 1, being **characterized in that** said non-tuberculosis mycobacteria being selected from the group consisting of: M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai and M. xenopi.

3. A chip for detection of Mycobacterium tuberculosis, non-tuberculosis mycobacteria and drug resistant of Mycobacterium tuberculosis, being **characterized in that** the chip comprises:
a solid matrix; and
a plurality of probes setting on said matrix, and said probes being selected from the group consisting of: SEQ ID NO: 3∼23, 26∼36.

4. The chip according to claim 3, being **characterized in that** said non-tuberculosis mycobacteria being selected from the group consisting of: M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai and M. xenopi.

5. A kit for detection of Mycobacterium tuberculosis, non-tuberculosis mycobacteria and drug resistant of Mycobacterium tuberculosis, being **characterized in that** the kit comprises a plurality of probes being selected from the group consisting of: SEQ ID NO: 3∼23, 26∼36.

6. The kit according to claim 5, being **characterized in that** said non-tuberculosis mycobacteria being selected from the group consisting of: M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai and M. xenopi.

7. The kit according to claim 5,
being **characterized in that** the kit comprises a solid matrix for setting said probes, preferably
being **characterized in that** said matrix is polystyrene.

8. The kit according to claim 5,
being **characterized in that** the kit comprises a primer set SEQ ID NO:1 and 2; or
being **characterized in that** the kit comprises a primer set SEQ ID NO: 24 and 25; or
being **characterized in that** the kit comprises a hybridization buffer for appropriate environment to perform hybridization reaction of said probes.

9. The kit according to claim 5, being **characterized in that** the kit comprises a wash buffer to remove residues which do not hybridize with said probes.

10. The kit according to claim 5, being **characterized in that** the kit comprising a streptavidin conjugate alkaline phosphatase to label hybridization products.

11. The kit according to claim 10, being **characterized in that** the kit comprises a dilution buffer for said streptavidin conjugate alkaline phosphatase to avoid non-specific binding with said hybridization products.

12. The kit according to claim 10,
being **characterized in that** the kit comprises a detection reagent to present color via interacting with said streptavidin conjugate alkaline phosphatase,
preferably being **characterized in that** the kit comprises a detection buffer to provide required environment for the reaction of color development.

13. A method for detection of Mycobacterium tuberculosis, non-tuberculosis mycobacteria and drug resistant of Mycobacterium tuberculosis, being **characterized in that** the method comprises following steps:
(a) amplifying 16S-23S rRNA ITS gene and rpoB gene from sample;
(b) providing a plurality of probes to hybridize product from step (a), wherein said probes being selected from the group consisting of: SEQ ID NO: 3∼23, 26∼36; and
(c) measuring result of hybridization reaction.

14. The method according to claim 13,
being **characterized in that** said non-tuberculosis mycobacteria being selected from the group consisting of: M. abscessus, M. asiaticum, M. avium, M. chelonae, M. fortuitum, M. gordonae, M. intracellulare, M. kansasii, M. lentiflavum, M. malmoense, M. marinum, M. scrofulaceum, M. shimodei, M. szulgai and M. xenopi; or
being **characterized in that** two primer sets SEQ ID NO: 1 and 2, 24 and 25 were used for amplification in step (a).

15. The method according to claim 13, being **characterized in that** the method also comprises the following step:
(d) spotting said probes on a solid matrix.
